# EUROPEAN PATENT SPECIFICATION

(11) **EP 4 403 924 B1**
(45) Date of publication and mention of the grant of the patent: **27.08.2025**
(21) Application number: 23214323.0
(22) Date of filing: 05.12.2023
(51) Int. Cl.: G01N 35/04, C12Q 1/00, G01N 35/10

(54) **TO-BE-TESTED OBJECT PROCESSING APPARATUS AND NUCLEIC ACID TESTING INTEGRATED MACHINE WITH TO-BE-TESTED OBJECT PROCESSING APPARATUS**
VORRICHTUNG ZUR VERARBEITUNG ZU TESTENDER OBJEKTE UND INTEGRIERTE NUKLEINSÄURETESTMASCHINE MIT VORRICHTUNG ZUR VERARBEITUNG ZU TESTENDER OBJEKTE
APPAREIL DE TRAITEMENT D'OBJET À TESTER ET MACHINE INTÉGRÉE DE TEST D'ACIDE NUCLÉIQUE AVEC APPAREIL DE TRAITEMENT D'OBJET À TESTER

(30) Priority: 19.01.2023 CN 202310101094
(43) Date of publication of application: 24.07.2024
(73) Proprietor: Shenzhen New Industries Biomedical Engineering Co., Ltd., Shenzhen, Guangdong 518122 (CN)
(72) Inventor: YIN, Li, Shenzhen, 518122 (CN); TANG, Junhui, Shenzhen, 518122 (CN); ZHU, Liang, Shenzhen, 518122 (CN); WANG, Yixian, Shenzhen, 518122 (CN); ZHENG, Xiaolin, Shenzhen, 518122 (CN); HE, Guoyao, Shenzhen, 518122 (CN); XIAO, Hao, Shenzhen, 518122 (CN)
(74) Representative: Studio Torta S.p.A.

(56) References cited:
- CA-A1- 3 162 684
- CN-A- 109 679 827
- US-A1- 2019 369 134

## Description

### Cross Reference to Related Applications

The present invention claims benefit of Chinese Patent Application No. 202310101094.0, filed on January 19, 2023, entitled "To-Be-Tested Object Processing Apparatus and Nucleic Acid Testing Integrated Machine with To-Be-Tested Object Processing Apparatus".

### Technical Field

The invention relates to the technical field of biological detection, in particular to a to-be-tested object processing apparatus and a nucleic acid testing integrated machine with the to-be-tested object processing apparatus.

### Background

At present, nucleic acid testing is widely used in clinical diagnosis, agricultural monitoring, food safety, and other fields, especially when a large-scale infectious disease occurs, nucleic acid testing can be used as the golden standard for disease diagnosis and judgment. Polymerase Chain Reaction (PCR) is the most commonly used method for nucleic acid testing. The general principle of PCR detection is a process of in vitro replication of daughter strand Deoxyribo Nucleic Acid (DNA) complementary to parent strand DNA with the parent strand DNA as a template and a specific primer as an extension starting point under the catalysis of DNA polymerase.

In the related technology, when extracting the parent strand DNA, a nucleic acid testing integrated machine requires the cooperation of a plurality of modules therein, and a movable gripper is used between the modules to transfer consumables, so as to extract the parent strand DNA from a sample by using an extraction reagent.

However, the transfer of the consumables between different modules needs to be realized by different grippers. Alternatively, the operation area of the gripper is arranged sufficiently large. The above two solutions require a large space, resulting in an increase in the volume of an apparatus.

The documents CN 109 679 827 A, CA 3 162 684 A1 and US 2019/369134 A1 disclose various apparatuses for processing an object to be tested, each comprising inter alia a frame body provided with a sample processing platform, an extraction mechanism and a grabbing part movably arranged above the sample processing platform in a vertical direction and a first horizontal direction.

### Summary

The invention provides a to-be-tested object processing apparatus and a nucleic acid testing integrated machine with the to-be-tested object processing apparatus, so as to solve the problem that the volume of the nucleic acid testing integrated machine in the related technology is large.

According to one aspect of the invention, a to-be-tested object processing apparatus is provided. The to-be-tested object processing apparatus includes: a frame body, provided with a sample processing platform; an extraction mechanism, configured to extract an analyte in a to-be-tested object; a grabbing part, movably arranged above the sample processing platform in a vertical direction and a first horizontal direction; a transfer part, movably arranged on the sample processing platform in a second horizontal direction perpendicular to the first horizontal direction, the transfer part including an extraction plate transfer part, the extraction plate transfer part being provided with a first transfer position where the extraction plate transfer part moving to below the grabbing part, the grabbing part being provided with a first grabbing position located above the extraction plate transfer part and a second grabbing position located above the extraction mechanism, and when the extraction plate transfer part is located in the first transfer position, the grabbing part being able to move between the first grabbing position and the second grabbing position to move an extraction plate between the extraction plate transfer part and the extraction mechanism; and a pipetting part, movably arranged above the sample processing platform. The extraction plate transfer part is further provided with a sample adding position corresponding to the pipetting part, and when the extraction plate transfer part is located in the sample adding position, the pipetting part is further provided with a first pipetting position where the pipetting part moving to above the extraction plate transfer part.

In some embodiments, the transfer part further includes an amplification plate transfer part. The extraction plate transfer part and the amplification plate transfer part move in the second horizontal direction, the amplification plate transfer part is provided with a nucleic acid adding position flush with the extraction plate transfer part, the pipetting part is further provided with a second pipetting position corresponding to an upper side of the amplification plate transfer part, and when the extraction plate transfer part is located in the sample adding position and the amplification plate transfer part is located in the nucleic acid adding position, the pipetting part is switched between the first pipetting position and the second pipetting position.

In some embodiments, the extraction mechanism includes an extraction part and a transport part movably penetrating through the extraction part in the second horizontal direction. The transport part is provided with a transport position corresponding to lower side of the grabbing part and an extraction position located below the extraction part, and when the extraction plate transfer part is located in the first transfer position and the transport part is located in the transport position, the grabbing part is able to move the extraction plate between the extraction plate transfer part and the transport part.

In some embodiments, the to-be-tested object processing apparatus further includes a film sealing mechanism configured to encapsulate an amplification plate, wherein the film sealing mechanism comprises a carrier and a hot pressing part configured to encapsulate the amplification plate. The hot pressing part is located above the carrier, the carrier is movably arranged on the sample processing platform in the second horizontal direction, and the carrier is provided with an amplification plate carrying part configured to place the amplification plate. The amplification plate transfer part is provided with a second transfer position located below the grabbing part. The grabbing part is further provided with a third grabbing position located above the carrier and a fourth grabbing position located above the amplification plate transfer part. When the amplification plate transfer part is located in the second transfer position, the grabbing part is able to move between the third grabbing position and the fourth grabbing position to move the amplification plate from the amplification plate transfer part to the amplification plate carrying part.

In some embodiments, the carrier is further provided with a cover body carrying part configured to place a cover body, when the grabbing part is located in the third grabbing position, the carrier is provided with a separation position where the cover body carrying part moves to below the grabbing part to make the grabbing part grab the cover body, a release position where the amplification plate carrying part moves to below the grabbing part to make the grabbing part release the cover body to the amplification plate, and an encapsulation position where the amplification plate carrying part moves to below the hot pressing part.

In some embodiments, the film sealing mechanism includes a film sealing frame and a detection piece. The film sealing frame is arranged on the sample processing platform, the hot pressing part and the detection piece are fixed to the film sealing frame, the carrier is movably arranged on the film sealing frame in the second horizontal direction, and the detection piece is able to detect a carrying state of the carrier.

In some embodiments, the cover body carrying part is provided with a first limiting protrusion configured to limit the cover body, and/or, the amplification plate carrying part is provided with a second limiting protrusion configured to limit the amplification plate.

In some embodiments, the to-be-tested object processing apparatus further includes a consumable storage bin configured to carry a pipette tip. The pipetting part is provided with a loading position where the pipetting part moving to above the consumable storage bin to load the pipette tip.

In some embodiments, the pipetting part includes a sample arm and a pipetting needle. The sample arm is horizontally movably arranged on the frame body and located above the sample processing platform, and the pipetting needle is vertically movably arranged on the sample arm.

In some embodiments, the frame body further includes a first guide rail and a second guide rail which are located above the sample processing platform. The first guide rail extends in the first horizontal direction, the second guide rail extends in the second horizontal direction, the first guide rail is movably arranged below the second guide rail in an extension direction of the second guide rail, and the sample arm is movably arranged on the first guide rail in an extension direction of the first guide rail.

In some embodiments, the grabbing part includes a grabbing frame and a gripper vertically movably arranged on the grabbing frame. The grabbing frame is movably arranged on the frame body in the first horizontal direction, and the gripper is provided with a first grabbing position and a second grabbing position.

According to another aspect of the invention, a nucleic acid testing integrated machine is provided. The nucleic acid testing integrated machine includes a reagent preparation apparatus, a to-be-tested object processing apparatus, and an amplification testing apparatus. The reagent preparation apparatus is configured to prepare an extraction reagent and an amplification reagent, the amplification testing apparatus is configured to perform analyte detection determination on a determination mixture to analyze a to-be-tested object, and the to-be-tested object processing apparatus is the above provided to-be-tested object processing apparatus.

Further, the reagent preparation apparatus, the to-be-tested object processing apparatus, and the amplification testing apparatus are isolated from each other, the reagent preparation apparatus and the amplification testing apparatus are respectively located on two sides of the to-be-tested object processing apparatus, a first channel is arranged between the reagent preparation apparatus and the to-be-tested object processing apparatus, a second channel is arranged between the to-be-tested object processing apparatus and the amplification testing apparatus, and the first channel and the second channel have an on state and an off state; and/or, the nucleic acid testing integrated machine further includes a ferry mechanism arranged between the reagent preparation device and the to-be-tested object processing apparatus. The ferry mechanism is able to move an extraction plate configured to prepare the extraction reagent, an amplification plate configured to prepare the amplification reagent, and a cover body to the to-be-tested object processing apparatus through the first channel, and agrabbing part of the to-be-tested object processing apparatus is able to move the extraction plate to an extraction plate transfer part of the to-be-tested object processing apparatus and move the amplification plate to an amplification plate transfer part of the to-be-tested object processing apparatus.

By applying the technical solutions of the invention, the to-be-tested object processing apparatus includes the frame body, the extraction mechanism, the grabbing part, the transfer part, and the pipetting part, when extracting the analyte in the to-be-tested object, the to-be-tested object is sucked by using the pipetting part, then the extraction plate transfer part moves to the sample adding position, and the pipetting part moves to the first pipetting position, so that the pipetting part releases the to-be-tested object into the extraction plate of the extraction plate transfer part. Then the extraction plate transfer part moves to the first transfer position, and the grabbing part moves to the first grabbing position above the extraction plate transfer part to grab the extraction plate on the extraction plate transfer part by using the grabbing part. Then the grabbing part moves to the second grabbing position located above the extraction mechanism to release the extraction plate to the extraction mechanism, and then the analyte in the to-be-tested object is extracted by using the extraction mechanism. Because the grabbing part moves in the vertical direction and the first horizontal direction, and the extraction plate transfer part moves in the second horizontal direction, the extraction of the analyte is realized, which greatly reduces the space occupation of the to-be-tested object processing apparatus, thereby reducing the volume of the to-be-tested object processing apparatus.

### Brief Description of the Drawings

The accompanying drawings of the specification, which constitute a part of the invention, are intended to provide a further understanding of the invention, and the exemplary embodiments of the invention and the description thereof are intended to explain the invention and do not constitute an undue limitation on the invention. In the accompanying drawings:
Fig. 1 shows a schematic structural diagram of a to-be-tested object processing apparatus according to an embodiment of the invention.
Fig. 2 shows a schematic structural diagram of a to-be-tested object processing apparatus in another angle according to an embodiment of the invention.
Fig. 3 shows a schematic structural diagram of an extraction mechanism in Fig. 1.
Fig. 4 shows a schematic structural diagram of a film sealing mechanism in Fig. 1.
Fig. 5 shows a schematic structural diagram of a pipetting part in Fig. 1.

Herein, the above accompanying drawings include the following reference signs:
10: Frame body; 11: Sample processing platform; 12: First guide rail; 13: Second guide rail;
20: To-be-tested object loading part;
30: Extraction mechanism; 31: Extraction part; 32: Transport part;
40: Grabbing part;
50: Transfer part; 53: Extraction plate transfer part; 54: Amplification plate transfer part;
60: Pipetting part; 61: Sample arm; 62: Pipetting needle;
70: Film sealing mechanism; 71: Hot pressing part; 72: Carrier; 721: Cover body carrying part; 722: Amplification plate carrying part; 73: Film sealing frame;
80: Consumable storage bin.

### Detailed Description of the Embodiments

The technical solutions in the embodiments of the invention will be clearly and completely described in conjunction with the accompanying drawings in the embodiments of the invention. It is apparent that the described embodiments are only a part of the embodiments of the invention, and not all of them. The following description of at least one exemplary embodiment is only illustrative, and in no way serves as any limitation on the invention or application or use thereof. All other embodiments obtained by those of ordinary skill in the art based on the embodiments of the invention without creative efforts are within the scope of protection of the invention.

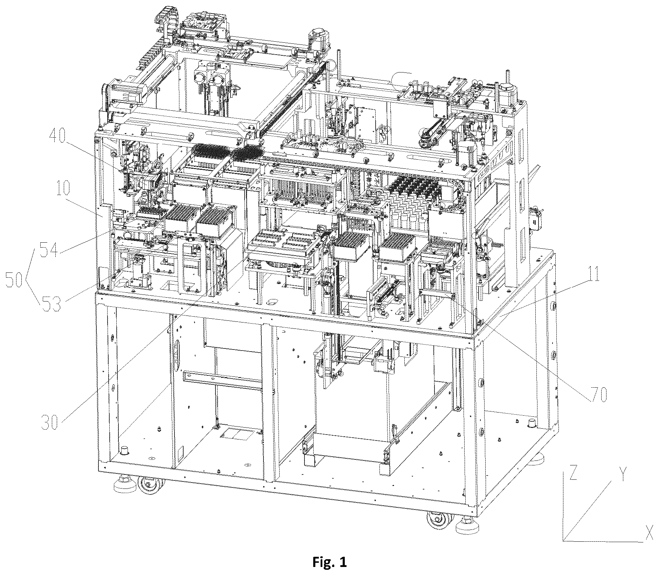

As shown in Fig. 1 to Fig. 5, the embodiments of the invention provide a to-be-tested object processing apparatus. The to-be-tested object processing apparatus includes: a frame body 10, an extraction mechanism 30, a grabbing part 40, a transfer part 50, and a pipetting part 60. The frame body 10 is provided with a sample processing platform 11. The extraction mechanism 30 is configured to extract an analyte in a to-be-tested object. The grabbing part 40 is movably arranged above the sample processing platform 11 in a vertical direction and a first horizontal direction. The transfer part 50 is movably arranged on the sample processing platform 11 in a second horizontal direction perpendicular to the first horizontal direction. The transfer part 50 includes an extraction plate transfer part 53, the extraction plate transfer part 53 being provided with a first transfer position where the extraction plate transfer part 53 moving to below the grabbing part 40. The grabbing part 40 is provided with a first grabbing position located above the extraction plate transfer part 53 and a second grabbing position located above the extraction mechanism 30, and when the extraction plate transfer part 53 is located in the first transfer position, the grabbing part 40 is able to move between the first grabbing position and the second grabbing position to move an extraction plate between the extraction plate transfer part 53 and the extraction mechanism 30. The pipetting part 60 is movably arranged above the sample processing platform 11. The extraction plate transfer part 53 is further provided with a sample adding position corresponding to the pipetting part 60, and when the extraction plate transfer part 53 is located in the sample adding position, the pipetting part 60 is further provided with a first pipetting position moving to above the extraction plate transfer part 53.

By applying the technical solutions of the invention, the to-be-tested object processing apparatus includes the frame body 10, the extraction mechanism 30, the grabbing part 40, the transfer part 50, and the pipetting part 60, when extracting the analyte in the to-be-tested object, the to-be-tested object is sucked by using the pipetting part 60 first, then the extraction plate transfer part 53 moves to the sample adding position, and the pipetting part 60 moves to the first pipetting position, so that the pipetting part 60 releases the to-be-tested object into the extraction plate of the extraction plate transfer part 53. Then the extraction plate transfer part 53 moves to the first transfer position, and the grabbing part 40 moves to the first grabbing position above the extraction plate transfer part 53 to grab the extraction plate on the extraction plate transfer part 53 by using the grabbing part 40. Then the grabbing part 40 moves to the second grabbing position located above the extraction mechanism 30 to release the extraction plate to the extraction mechanism 30, and then the analyte in the to-be-tested object is extracted by using the extraction mechanism 30. Because the grabbing part 40 moves in the vertical direction and the first horizontal direction, and the extraction plate transfer part 53 moves in the second horizontal direction, the extraction of the analyte is realized, which greatly reduces the space occupation of the to-be-tested object processing apparatus, thereby reducing the volume of the to-be-tested object processing apparatus.

It is to be noted that the to-be-tested object is transferred from the outside of the apparatus to the sample processing platform 11 in the apparatus, or a to-be-tested object loading part 20 is arranged on the sample processing platform 11, and the to-be-tested object is placed in the to-be-tested object loading part 20.

In the embodiment, the to-be-tested object loading part 20 includes a to-be-tested object bin configured to the to-be-tested object and a cover opening mechanism. The to-be-tested object is contained in a to-be-tested object tube, and the to-be-tested object tube is placed in the to-be-tested object bin. A cover is opened by a cover opening mechanism, and the to-be-tested object tube after the cover is opened is transferred to below the pipetting part 60 to suck the to-be-tested object.

It is to be noted that the to-be-tested object includes one or more of samples to be measured, quality control products or calibration products.

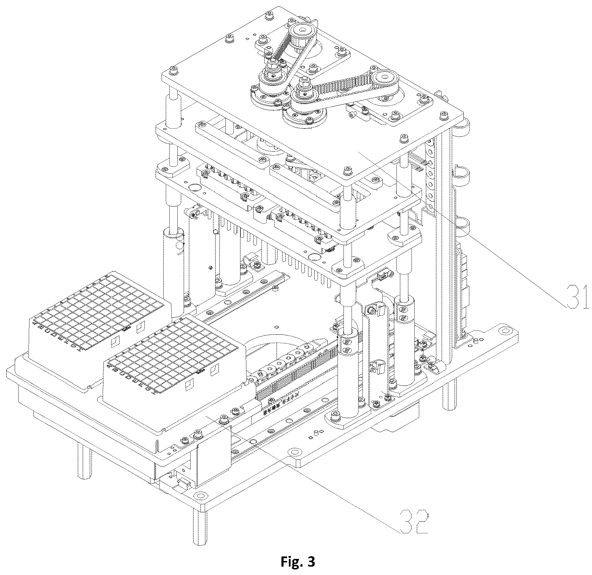

As shown in Fig. 1 and Fig. 3, the extraction mechanism 30 includes an extraction part 31 and a transport part 32 movably penetrating through the extraction part 31 in the second horizontal direction. The transport part 32 is provided with a transport position corresponding to a lower side of the grabbing part 40 and an extraction position located below the extraction part 31, and when the extraction plate transfer part 53 is located in the first transfer position and the transport part 32 is located in the transport position, the grabbing part 40 is able to move the extraction plate between the extraction plate transfer part 53 and the transport part 32. Through the adoption of the above structure, the transport part 32 is able to realize the transport of the extraction plate between the extraction plate transfer part 53 and the extraction part 31, which has the advantages of simple structure and easy realization.

In the embodiment, the extraction plate transfer part 53 and the transport part 32 are able to move to below the grabbing part 40 in the second horizontal direction. As shown in Fig. 1 to Fig. 3, the transfer part 50 further includes an amplification plate transfer part 54. The extraction plate transfer part 53 and the amplification plate transfer part 54 move in the second horizontal direction, the amplification plate transfer part 54 is provided with a nucleic acid adding position flush with the extraction plate transfer part 53, the pipetting part 60 is further provided with a second pipetting position corresponding to an upper side of the amplification plate transfer part 54, and when the extraction plate transfer part 53 is located in the sample adding position and the amplification plate transfer part 54 is located in the nucleic acid adding position, the pipetting part 60 is switched between the first pipetting position and the second pipetting position. When the amplification plate transfer part 54 is located in the nucleic acid adding position and the extraction plate transfer part 53 is located in the sampling adding position, the extracted nucleic acid is filled from the extraction plate located on the extraction plate transfer part 53 to the amplification plate located on the amplification plate transfer part 54 by using the pipetting part 60, which has the advantages of compact structure and easy operation.

In the embodiment, the extraction plate transfer part 53 and the amplification plate transfer part 54 are arranged in parallel, two parallel tracks are arranged on the sample processing platform 11, both of which extend in the second horizontal direction, and the extraction plate transfer part 53 and the amplification plate transfer part 54 are respectively movably arranged on the corresponding tracks.

It is to be noted that after the analyte of the to-be-tested object in the extraction plate is extracted in the extraction mechanism 30, the extraction plate is grabbed by using the grabbing part 40 and released to the extraction plate transfer part 53 located in the first transfer position. Then the extraction plate transfer part 53 moves to the sample adding position, the pipetting part 60 moves to the first pipetting position to suck the extracted analyte in the extraction plate, and the pipetting part 60 moves to the second pipetting position located above the amplification plate transfer part 54 to fill the analyte into the amplification plate, thereby completing the transfer of the analyte.

The amplification plate transfer part 54 is further provided with a second transfer position corresponding to a lowerside of the grabbing part 40, so that the grabbing part 40 loads the amplification plate.

The grabbing part 40 moves in the vertical direction and the first horizontal direction to realize the transfer of the extraction plate between the extraction plate transfer part 53 and the extraction mechanism 30. The extraction plate transfer part 53, the amplification plate transfer part 54, and the transport part 32 moves in the second horizontal direction, so that the to-be-tested object is sucked by the pipetting part 60 and then transferred to the extraction part 31 for the extraction of the analyte and the extracted analyte is transferred from the extraction plate to the amplification plate, which makes the arrangement of each portion more reasonable and compact to reduce the volume of the apparatus.

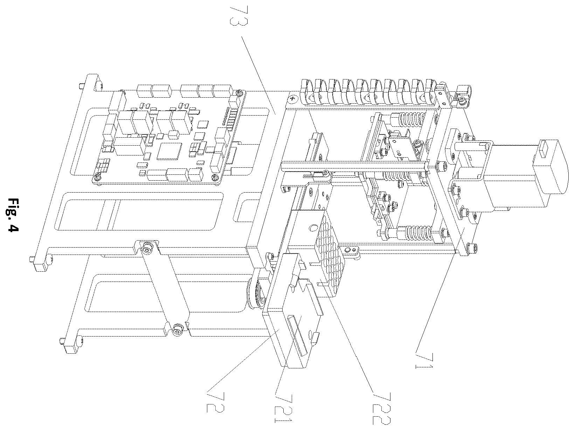

As shown in Fig. 1 and Fig. 4, the to-be-tested object processing apparatus further includes a film sealing mechanism 70 configured to encapsulate an amplification plate. The film sealing mechanism 70 includes a carrier 72 and a hot pressing part 71 configured to encapsulate the amplification plate. The hot pressing part 71 is located above the carrier 72, the carrier 72 is movably arranged on the sample processing platform 11 in the second horizontal direction, the carrier 72 is provided with an amplification plate carrying part 722 configured to place the amplification plate, and the amplification plate carrying part 722 is provided with a third transfer position correspondingly moving to below the grabbing part 40. The amplification plate transfer part 54 is provided with a second transfer position located below the grabbing part 40. The grabbing part 40 is further provided with a third grabbing position located above the carrier 72 and a fourth grabbing position located above the amplification plate transfer part 54. When the amplification plate transfer part 54 is located in the second transfer position and the amplification plate carrying part 722 is located in the third transfer position, the grabbing part 40 is able to move between the third grabbing position and the fourth grabbing position to move the amplification plate from the amplification plate transfer part 54 to the amplification plate carrying part 722. Through the adoption of the above structure, the carrier 72 is able to carry the amplification plate, and the grabbing part 40 moves the amplification plate loaded with the analyte to the carrier 72, so that the encapsulation of the amplification plate is completed by using the hot pressing part 71.

In addition, the extraction plate and the amplification plate are transferred by using the grabbing part 40. In the transfer process of the extraction plate and the amplification plate, the extraction plate and the amplification plate have a shared moving path, which is conducive to reducing the space occupation, thereby making the structure of the to-be-tested object processing apparatus more compact.

As shown in Fig. 4, the carrier 72 is further provided with a cover body carrying part 721 configured to place a cover body, when the grabbing part 40 is located in the third grabbing position, the carrier 72is provided with a separation position where the cover body carrying part 721 moves to below the grabbing part 40 to make the grabbing part 40 grab the cover body, a release position where the amplification plate carrying part 722 moves to below the grabbing part 40 to make the grabbing part40 release the cover body to the amplification plate, and an encapsulation position where the amplification plate carrying part 722 moves to below the hot pressing part 71. The carrier 72 moves in the second horizontal direction within the motion range of the grabbing part 40 to cooperate with the grabbing part 40, so that the encapsulation of the cover body and the amplification plate is realized, which has the advantage of compact structure.

Specifically, when the grabbing part is located in the fourth grabbing position, the carrier 72 moves to the separation position, and then the grabbing part 40 is able to grab the cover body and remove it from the cover body carrying part 721. Then the carrier 72 moves to the release position in the second horizontal direction, so that the amplification plate carrier 722 moves to below the grabbing part 40, so as to release the cover body to the amplification plate. Then the carrier 72 moves the encapsulation position, and the cover body and the amplification plate are encapsulated by the hot pressing part 71.

As shown in Fig. 4, the film sealing mechanism 70 includes a film sealing frame 73 and a detection piece. The film sealing frame 73 is arranged on the sample processing platform 11, the hot pressing part 71 and the detection piece are fixed to the film sealing frame 73, the carrier 72 is movably arranged on the film sealing frame 73 in the second horizontal direction, and the detection piece is able to detect a carrying state of the carrier 72. The detection piece is able to detect whether the amplification plate and the cover body or the encapsulated amplification plate and cover body are arranged on the carrier 72.

In the embodiment, the cover body carrying part 721 is provided with a first limiting protrusion configured to limit the cover body, and the amplification plate carrying part 722 is provided with a second limiting protrusion configured to limit the amplification plate. The cover body is limited by using the first limiting protrusion, so that the cover body is more stable on the cover body carrying part 721. The amplification plate is limited by using the second limiting protrusion, so that the amplification plate is more stable on the amplification plate carrying part 722.

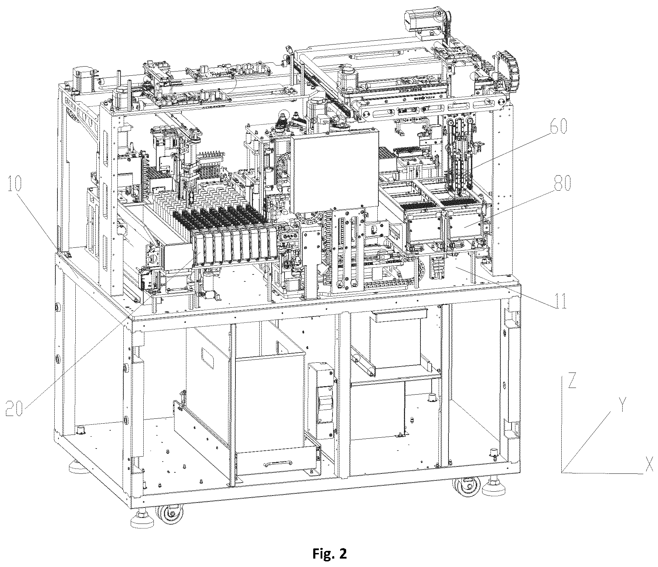

As shown in Fig. 2, the to-be-tested object processing apparatus further includes a consumable storage bin 80 configured to carry a pipette tip. The pipetting part 60 is provided with a loading position where the pipetting part 60moving to above the consumable storage bin 80 to load the pipette tip. The pipette tip is stored by using the consumable storage bin 80, and the pipette tip is loaded by using the pipetting part 60, and replaced before the pipette tip sucks the to-be-tested object or the analyte to prevent contamination between the extraction reagent, the amplification reagent, the to-be-tested object, and the analyte.

Specifically, the pipetting part 60 needs to move to above the consumable storage bin 80 to load the pipette tip before the pipetting part 60 sucks the to-be-tested object in the to-be-tested object tube. Then the to-be-tested object is sucked by using the pipette tip. The process of sucking the analyte is similar to the above, and will not be elaborated herein.

As shown in Fig. 1, the pipetting part 60 includes a sample arm 61 and a pipetting needle 62. The sample arm 61 is horizontally movably arranged on the frame body 10 and located above the sample processing platform 11, and the pipetting needle 62 is vertically movably arranged on the sample arm 61. The pipetting part 60 adopting the above structure is able to realize the three-axis movement of the pipetting needle 62 by using the cooperation of the sample arm 61 and the pipetting needle 62.

In the embodiment, the pipette tip is loaded by the pipetting needle 62. Of course, a plurality of pipetting needles 62 are also arranged on the sample arm 61, and in order to prevent contamination, pipetting needles 62 are used for each operation.

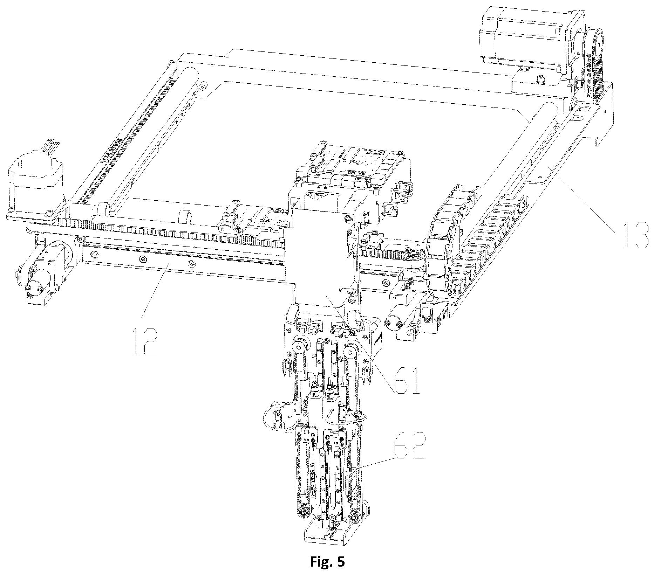

As shown in Fig. 1 and Fig. 5, the frame body 10 further includes a first guide rail 12 and a second guide rail 13which are located above the sample processing platform 11. The first guide rail 12 extends in the first horizontal direction, the second guide rail 13 extends in the second horizontal direction, the first guide rail 12 is movably arranged above the second guide rail 13 in an extension direction of the second guide rail 13, and the sample arm 61 is movably arranged on the first guide rail 12 in an extension direction of the first guide rail 12. The movement of the sample arm 61 in the horizontal direction is realized by using the first guide rail 12 and the second guide rail 13, which has the advantage of simple structure.

As shown in Fig. 5, the grabbing part 40 includes a grabbing frame and a gripper vertically movably arranged on the grabbing frame. The grabbing frame is movably arranged on the frame body 10 in the first horizontal direction, and the gripper is provided with a first grabbing position and a second grabbing position. The grabbing part 40 adopting the above structure is able to realize the two-axis movement of the gripper in a vertical plane by using the grabbing frame, which has the advantage of simple structure.

The following is described in conjunction with the use process of the to-be-tested object processing apparatus. When extracting the analyte in the to-be-tested object, the to-be-tested object is sucked by using the pipetting needle 62 on the pipetting part 60 first, then the pipetting needle 62 moves to the first pipetting position located above the extraction plate transfer part 53, and the extraction plate transfer part 53 moves to the sample adding position, so that the pipetting needle 62 releases the to-be-tested object into the extraction plate of the extraction plate transfer part 53. Then the extraction plate transfer part 53 moves to the first transfer position located below the grabbing part 40, and the grabbing part 40 moves to the first grabbing position to grab the extraction plate on the extraction plate transfer part 53 by using the grabbing part 40. Then the grabbing part 40 moves to the second grabbing position to be located above the transport part 32 located in the transport position, so as to release the extraction plate to the transport part 32, and the transport part 32 moves to the extraction position located in the extraction part 31, so that the analyte in the to-be-tested object is extracted by using the extraction part 31. After extracting the analyte, the transport part 32 moves from the extraction position to the transport position, and the extraction plate is grabbed by using the grabbing part 40 and released to the extraction plate transfer part 53 located in the first transfer position. Then, the extraction plate transfer part 53 moves to the sample adding position, the pipetting needle 62 moves to the first pipetting position, and the extracted analyte in the extraction plate is sucked by using the pipetting needle 62. Then the pipetting needle 62 moves to the second pipetting position to be located above the amplification plate transfer part 54 located in the nucleic acid adding position, so as to release the analyte into the amplification plate. Then the amplification plate transfer part 54 moves to the second transfer position, the grabbing part 40 moves to the fourth grabbing position, the amplification plate is grabbed by using the grabbing part 40, and the grabbing part 40 moves to the third grabbing position located above the carrier 72 to release the amplification plate to the amplification plate carrying part 722. The carrier 72 moves to the separation position, the cover body is grabbed by using the grabbing part 40 and the carrier 72 is released to the release position, so as to release the cover body to the amplification plate, and then the carrier 72 moves to the encapsulation position to encapsulate the cover body and the amplification plate by using the hot pressing part 71. Therefore, the transfer of the extraction plate, the amplification plate, and the cover body in the same path, as well as the combination of the amplification plate and the cover body are realized through the to-be-tested object processing apparatus, which has the characteristic of simple structure and reduces the volume of the to-be-tested object processing apparatus.

Another embodiment of the invention provides a nucleic acid testing integrated machine. The nucleic acid testing integrated machine includes a reagent preparation apparatus, a to-be-tested object processing apparatus, and an amplification testing apparatus. The reagent preparation apparatus is configured to prepare an extraction reagent and an amplification reagent, the amplification testing apparatus is configured to perform analyte detection determination on a determination mixture to analyze a to-be-tested object, and the to-be-tested object processing apparatus is the above provided to-be-tested object processing apparatus. Because agrabbing part 40 moves in the vertical direction and a first horizontal direction, and a transfer part 50 moves in the second horizontal direction, the extraction of an analyte is realized, which greatly reduces the space occupation of the to-be-tested object processing apparatus, thereby reducing the volume of the to-be-tested object processing apparatus.

In the embodiment, the reagent preparation apparatus, the to-be-tested object processing apparatus, and the amplification testing apparatus are isolated from each other, the reagent preparation apparatus and the amplification testing apparatus are respectively located on two sides of the to-be-tested object processing apparatus, a first channel is arranged between the reagent preparation apparatus and the to-be-tested object processing apparatus, a second channel is arranged between the to-be-tested object processing apparatus and the amplification testing apparatus, and the first channel and the second channel have an on state and an off state. Through the adoption of the above structure, isolation between modules of the apparatus is realized by closing the first channel and the second channel, thereby avoiding cross contamination. The transmission of consumables between the modules of the apparatus is realized by the communication of the first channel and the second channel.

The nucleic acid testing integrated machine further includes a ferry mechanism arranged between the reagent preparation device and the to-be-tested object processing apparatus. The ferry mechanism is able to move an extraction plate configured to prepare the extraction reagent, an amplification plate configured to prepare the amplification reagent, and a cover body to the to-be-tested object processing apparatus through the first channel, and the grabbing part 40 of the to-be-tested object processing apparatus is able to move the extraction plate to an extraction plate transfer part 53 of the to-be-tested object processing apparatus and move the amplification plate to an amplification plate transfer part 54 of the to-be-tested object processing apparatus. The ferry mechanism is able to move the extraction plate that completes the preparation of the extraction reagent and the amplification plate that completes the preparation of the amplification reagent in the reagent preparation apparatus to the to-be-tested object processing apparatus for the extraction of the to-be-tested object.

In the embodiment, the extraction plate, the amplification plate, and the cover body are transferred from the reagent preparation apparatus to the to-be-tested object processing apparatus through the ferry mechanism, the extraction plate, amplification plate and cover body are transferred respectively by using the grabbing part 40, the extraction plate is placed on the extraction plate transfer part 53, the amplification plate is placed on the amplification plate transfer part 54, the cover body is placed on a cover body carrying part 721 of a carrier 72, and the ferry mechanism is arranged within the motion range of the grabbing part 40. Therefore, the transfer paths of the grabbing part 40 transferring the extraction plate from the ferry mechanism to the extraction plate transfer part, transferring the amplification plate from the ferry mechanism to the amplification plate transfer part, and transferring the cover body from the ferry mechanism to the carrier 72 are overlapped, so that the structure is more compact.

A running track of the ferry mechanism, a running track of the transfer part 50, a running track of a transport part 32, and a running track of the carrier 72 are arranged below the grabbing part 40 in sequence in the moving direction of the grabbing part 40, so that the structure is more compact.

It is to be noted that the terms used herein is only for the purpose of describing the specific implementation modes and is not intended to limit the exemplary implementation modes of the invention. As used herein, the singular form is also intended to include the plural form unless otherwise expressly stated in the context, and it should also be understood that when the terms "contain" and/or "include" are used in the specification, they indicate the presence of features, steps, operations, devices, components and/or combinations thereof.

Unless otherwise specified, the relative arrangement, numerical expressions and numerical values of parts and steps set forth in these embodiments do not limit the scope of the invention. Also, it should be understood that, for ease of description, the dimensions of various parts shown in the accompanying drawings are not drawn to scale. Techniques, methods, and devices known to those of ordinary skill in the related fields may not be discussed in detail, but should be regarded as part of the specification under appropriate circumstances. In all examples shown and discussed herein, any specific value should be interpreted as exemplary only and not as a limitation. Thus, other examples of the exemplary embodiments may have different values. It is to be noted that: similar numbers and letters refer to similar items in the following accompanying drawings, and thus, once an item is defined in one accompanying drawing, it does not require further discussion in subsequent accompanying drawings.

In the description of the invention, it is to be understood that the orientations or positional relationships indicated by the orientation words "front, rear, upper, down, left and right", "transverse, longitudinal, vertical and horizontal", "top and bottom", etc. are based on the orientations or positional relationships shown in the accompanying drawings, and are only for the convenience of describing the invention and simplifying the description. Unless stated to the contrary, these orientation words do not indicate or imply that the apparatus or element referred to must have a specific orientation, be constructed and operated in a specific orientation, and therefore cannot be construed as limiting the scope of protection of the invention. The orientation words "inside and outside" refer to inside and outside relative to the outline of each part itself.

For ease of description, spatially relative terms, such as "over", "above", "on the surface", "upper", etc., may be used herein to describe the spatial positional relationship between a device or feature and other devices or features as shown in the figures. It should be understood that the spatially relative terms are intended to include different orientations of the device in use or operation in addition to the orientation described in the figures. For example, if the devices in the accompanying drawings are inverted, those described as "above other devices or structures" or "over other devices or structures" will then be positioned as "below other devices or structures" or "under other devices or structures". Thus, the exemplary term "above" may include both "above" and "below" orientations. The device may also be positioned in various other ways (rotated 90 degrees or at other orientations) and the spatially relative descriptions used herein are interpreted accordingly.

Furthermore, it is to be noted that the use of the words "first", "second" and the like to define parts is only for the convenience of distinguishing the corresponding parts, unless otherwise stated, the words have no special meaning, and therefore cannot be construed as limiting the scope of protection of the invention.

The above is only the preferred embodiments of the invention, and is not intended to limit the invention, and for those of ordinary skill in the art, various modifications and changes may be made to the invention.

## Claims

1. A to-be-tested object processing apparatus, comprising:
a frame body (10), provided with a sample processing platform (11);
an extraction mechanism (30), configured to extract an analyte in a to-be-tested object;
a grabbing part (40), movably arranged above the sample processing platform (11) in a vertical direction and a first horizontal direction;
a transfer part (50), movably arranged on the sample processing platform (11) in a second horizontal direction perpendicular to the first horizontal direction, the transfer part (50) comprising an extraction plate transfer part (53), the extraction plate transfer part (53) being provided with a first transfer position where the extraction plate transfer part (53)moving to below the grabbing part (40), the grabbing part (40) being provided with a first grabbing position located above the extraction plate transfer part (53) and a second grabbing position located above the extraction mechanism (30), and when the extraction plate transfer part (53) is located in the first transfer position, the grabbing part (40) being able to move between the first grabbing position and the second grabbing position to move an extraction plate between the extraction plate transfer part (53) and the extraction mechanism (30); and
a pipetting part (60), movably arranged above the sample processing platform (11);
wherein the extraction plate transfer part (53) is further provided with a sample adding position corresponding to the pipetting part (60), and when the extraction plate transfer part (53) is located in the sample adding position, the pipetting part (60) is further provided with a first pipetting position where the pipetting part (60)moving to above the extraction plate transfer part (53).

2. The to-be-tested object processing apparatus according to claim 1, wherein the transfer part (50) further comprises an amplification plate transfer part (54), wherein the extraction plate transfer part (53) and the amplification plate transfer part (54) move in the second horizontal direction, the amplification plate transfer part (54) is provided with a nucleic acid adding position flush with the extraction plate transfer part (53), the pipetting part (60) is further provided with a second pipetting position corresponding to an upper side of the amplification plate transfer part (54), and when the extraction plate transfer part (53) is located in the sample adding position and the amplification plate transfer part (54) is located in the nucleic acid adding position, the pipetting part (60) is switched between the first pipetting position and the second pipetting position.

3. The to-be-tested object processing apparatus according to claim 2, wherein the extraction mechanism (30) comprises an extraction part (31) and a transport part (32) movably penetrating through the extraction part (31) in the second horizontal direction, wherein the transport part (32) is provided with a transport position corresponding to a lower side of the grabbing part (40) and an extraction position located below the extraction part (31), and when the extraction plate transfer part (53) is located in the first transfer position and the transport part (32) is located in the transport position, the grabbing part (40) is able to move the extraction plate between the extraction plate transfer part (53) and the transport part (32).

4. The to-be-tested object processing apparatus according to claim 2, further comprising a film sealing mechanism (70) configured to encapsulate an amplification plate, wherein the film sealing mechanism (70) comprises a carrier (72) and a hot pressing part (71) configured to encapsulate the amplification plate, wherein the hot pressing part (71) is located above the carrier (72), the carrier (72) is movably arranged on the sample processing platform (11) in the second horizontal direction, and the carrier (72) is provided with an amplification plate carrying part (722) configured to place the amplification plate; the amplification plate transfer part (54) is provided with a second transfer position located below the grabbing part (40); the grabbing part (40) is further provided with a third grabbing position located above the carrier (72) and a fourth grabbing position located above the amplification plate transfer part (54); and when the amplification plate transfer part (54) is located in the second transfer position, the grabbing part (40) is able to move between the third grabbing position and the fourth grabbing position to move the amplification plate from the amplification plate transfer part (54) to the amplification plate carrying part (722).

5. The to-be-tested object processing apparatus according to claim 4, wherein the carrier (72) is further provided with a cover body carrying part (721) configured to place a cover body, when the grabbing part (40) is located in the third grabbing position, the carrier (72) is provided with a separation position where the cover body carrying part (721) moves to below the grabbing part (40) to make the grabbing part (40) grab the cover body, a release position where the amplification plate carrying part (722) moves to below the grabbing part (40) to make the grabbing part (40) release the cover body to the amplification plate, and an encapsulation position where the amplification plate carrying part (722) moves to below the hot pressing part (71).

6. The to-be-tested object processing apparatus according to claim 5, wherein the film sealing mechanism (70) comprises a film sealing frame (73) and a detection piece, wherein the film sealing frame (73) is arranged on the sample processing platform (11), the hot pressing part (71) and the detection piece are fixed to the film sealing frame (73), the carrier (72) is movably arranged on the film sealing frame (73) in the second horizontal direction, and the detection piece is able to detect a carrying state of the carrier (72).

7. The to-be-tested object processing apparatus according to claim 5, wherein the cover body carrying part (721) is provided with a first limiting protrusion configured to limit the cover body, and/or, the amplification plate carrying part (722) is provided with a second limiting protrusion configured to limit the amplification plate.

8. The to-be-tested object processing apparatus according to claim 1, further comprising a consumable storage bin (80) configured to carry a pipette tip, wherein the pipetting part (60) is provided with a loading position where the pipetting part (60)moving to above the consumable storage bin (80) to load the pipette tip.

9. The to-be-tested object processing apparatus according to claim 1, wherein the pipetting part (60) comprises a sample arm (61) and a pipetting needle (62), wherein the sample arm (61) is horizontally movably arranged on the frame body (10) and located above the sample processing platform (11), and the pipetting needle (62) is vertically movably arranged on the sample arm (61).

10. The to-be-tested object processing apparatus according to claim 9, wherein the frame body (10) is provided with a first guide rail (12) and a second guide rail (13) which are located above the sample processing platform (11), the first guide rail (12) extends in the first horizontal direction, the second guide rail (13) extends in the second horizontal direction, the first guide rail (12) is movably arranged below the second guide rail (13) in an extension direction of the second guide rail (13), and the sample arm (61) is movably arranged on the first guide rail (12) in an extension direction of the first guide rail (12).

11. The to-be-tested object processing apparatus according to claim 1, wherein the grabbing part (40) comprises a grabbing frame and a gripper vertically movably arranged on the grabbing frame, wherein the grabbing frame is movably arranged on the frame body (10) in the first horizontal direction, and the gripper is provided with a first grabbing position and a second grabbing position.

12. A nucleic acid testing integrated machine, comprising a reagent preparation apparatus, a to-be-tested object processing apparatus, and an amplification testing apparatus, wherein the reagent preparation apparatus is configured to prepare an extraction reagent and an amplification reagent, the amplification testing apparatus is configured to perform analyte detection determination on a determination mixture to analyze a to-be-tested object, and the to-be-tested object processing apparatus is the to-be-tested object processing apparatus according to any one of claims 1-11.

13. The nucleic acid testing integrated machine according to claim 12, wherein,
the reagent preparation apparatus, the to-be-tested object processing apparatus, and the amplification testing apparatus are isolated from each other, the reagent preparation apparatus and the amplification testing apparatus are respectively located on two sides of the to-be-tested object processing apparatus, a first channel is arranged between the reagent preparation apparatus and the to-be-tested object processing apparatus, a second channel is arranged between the to-be-tested object processing apparatus and the amplification testing apparatus, and the first channel and the second channel have an on state and an off state; and/or,
the nucleic acid testing integrated machine further comprises a ferry mechanism arranged between the reagent preparation device and the to-be-tested object processing apparatus, wherein the ferry mechanism is able to move an extraction plate configured to prepare the extraction reagent, an amplification plate configured to prepare the amplification reagent, and a cover body to the to-be-tested object processing apparatus through the first channel, and the grabbing part (40) of the to-be-tested object processing apparatus is able to move the extraction plate to an extraction plate transfer part (53) of the to-be-tested object processing apparatus and move the amplification plate to an amplification plate transfer part (54) of the to-be-tested object processing apparatus.

## Patentansprüche

1. Vorrichtung zur Verarbeitung von zu testenden Objekten, die Folgendes umfasst:
einen Rahmenkörper (10), der mit einer Probenverarbeitungsplattform (11) versehen ist;
einen Extraktionsmechanismus (30), der so konfiguriert ist, dass er einen Analyten in einem zu testenden Objekt extrahiert;
ein Greifteil (40), das oberhalb der Probenverarbeitungsplattform (11) in einer vertikalen Richtung und einer ersten horizontalen Richtung beweglich angeordnet ist;
ein Transferteil (50), das auf der Probenverarbeitungsplattform (11) in einer zweiten horizontalen Richtung senkrecht zu der ersten horizontalen Richtung beweglich angeordnet ist, wobei das Transferteil (50) ein Extraktionsplattentransferteil (53) umfasst, wobei das Extraktionsplattentransferteil (53) mit einer ersten Transferposition versehen ist, in der sich das Extraktionsplattentransferteil (53) nach unterhalb des Greifteils (40) bewegt, wobei das Greifteil (40) mit einer ersten Greifposition, die sich oberhalb des Extraktionsplattentransferteils (53) befindet, und einer zweiten Greifposition, die sich oberhalb des Extraktionsmechanismus (30) befindet, versehen ist, und wenn sich das Extraktionsplattentransferteil (53) in der ersten Transferposition befindet, das Greifteil (40) in der Lage ist, sich zwischen der ersten Greifposition und der zweiten Greifposition zu bewegen, um eine Extraktionsplatte zwischen dem Extraktionsplattentransferteil (53) und dem Extraktionsmechanismus (30) zu bewegen; und
ein Pipettierteil (60), das beweglich oberhalb der Probenverarbeitungsplattform (11) angeordnet ist;
wobei der Extraktionsplattentransferteil (53) ferner mit einer Probenzugabeposition versehen ist, die dem Pipettierteil (60) entspricht, und wenn sich der Extraktionsplattentransferteil (53) in der Probenzugabeposition befindet, das Pipettierteil (60) ferner mit einer ersten Pipettierposition versehen ist, in der sich das Pipettierteil (60) nach oberhalb des Extraktionsplattentransferteils (53) bewegt.

2. Vorrichtung zur Verarbeitung von zu testenden Objekten nach Anspruch 1, wobei das Transferteil (50) ferner ein Amplifikationsplattentransferteil (54) umfasst, wobei sich das Extraktionsplattentransferteil (53) und das Amplifikationsplattentransferteil (54) in der zweiten horizontalen Richtung bewegen, wobei das Amplifikationsplattentransferteil (54) mit einer Nukleinsäurezugabeposition versehen ist, die mit dem Extraktionsplattentransferteil (53) fluchtet, das Pipettierteil (60) ferner mit einer zweiten Pipettierposition versehen ist, die einer Oberseite des Amplifikationsplattentransferteils (54) entspricht, und wenn sich das Extraktionsplattentransferteil (53) in der Probenzugabeposition befindet und sich das Amplifikationsplattentransferteil (54) in der Nukleinsäurezugabeposition befindet, wird das Pipettierteil (60) zwischen der ersten Pipettierposition und der zweiten Pipettierposition umgeschaltet.

3. Vorrichtung zur Verarbeitung von zu testenden Objekten nach Anspruch 2, wobei der Extraktionsmechanismus (30) ein Extraktionsteil (31) und ein Transportteil (32) umfasst, das das Extraktionsteil (31) in der zweiten horizontalen Richtung beweglich durchdringt, wobei das Transportteil (32) mit einer Transportposition, die einer unteren Seite des Greifteil s (40) entspricht, und einer Extraktionsposition, die sich unterhalb des Extraktionsteils (31) befindet, versehen ist, und wenn sich das Extraktionsplattentransferteil (53) in der ersten Transferposition befindet und sich das Transportteil (32) in der Transportposition befindet, ist das Greifteil (40) in der Lage, die Extraktionsplatte zwischen dem Extraktionsplattentransferteil (53) und dem Transportteil (32) zu bewegen.

4. Vorrichtung zur Verarbeitung von zu testenden Objekten nach Anspruch 2, die ferner einen Folienversiegelungsmechanismus (70) umfasst, der so konfiguriert ist, dass er eine Amplifikationsplatte einkapselt, wobei der Folienversiegelungsmechanismus (70) einen Träger (72) und ein Heißpressteil (71) umfasst, das so konfiguriert ist, dass es die Amplifikationsplatte einkapselt, wobei sich das Heißpressteil (71) oberhalb des Trägers (72) befindet, der Träger (72) beweglich auf der Probenverarbeitungsplattform (11) in der zweiten horizontalen Richtung angeordnet ist und der Träger (72) mit einem Amplifikationsplattentrageteil (722) versehen ist, das zum Platzieren der Amplifikationsplatte konfiguriert ist; der Amplifikationsplattentransferteil (54) ist mit einer zweiten Transferposition versehen, die sich unterhalb des Greifteils (40) befindet; das Greifteil (40) ist ferner mit einer dritten Greifposition, die sich oberhalb des Trägers (72) befindet, und einer vierten Greifposition versehen, die sich oberhalb des Amplifikationsplattentransferteils (54) befindet; und wenn sich das Amplifikationsplattentransferteil (54) in der zweiten Transferposition befindet, ist das Greifteil (40) in der Lage, sich zwischen der dritten Greifposition und der vierten Greifposition zu bewegen, um die Amplifikationsplatte von dem Amplifikationsplattentransferteil (54) zu dem Amplifikationsplattentrageteil (722) zu bewegen.

5. Vorrichtung zur Verarbeitung von zu testenden Objekten nach Anspruch 4, wobei der Träger (72) ferner mit einem Abdeckkörpertrageteil (721) versehen ist, das dazu konfiguriert ist, einen Abdeckkörper zu platzieren, wenn sich das Greifteil (40) in der dritten Greifposition befindet, der Träger (72) mit einer Trennposition, in der sich das Abdeckkörpertrageteil (721) unter das Greifteil (40) bewegt, um das Greifteil (40) dazu zu bringen, den Abdeckkörper zu greifen, einer Freigabeposition, in der sich das Amplifikationsplattentrageteil (722) unter das Greifteil (40) bewegt, um das Greifteil (40) dazu zu bringen, den Abdeckkörper an die Amplifikationsplatte freizugeben, und einer Verkapselungsposition versehen ist, in der sich das Amplifikationsplattentrageteil (722) unter das Heißpressteil (71) bewegt.

6. Vorrichtung zur Verarbeitung von zu testenden Objekten nach Anspruch 5, wobei der Folienversiegelungsmechanismus (70) einen Folienversiegelungsrahmen (73) und ein Erfassungsstück umfasst, wobei der Folienversiegelungsrahmen (73) auf der Probenverarbeitungsplattform (11) angeordnet ist, das Heißpressteil (71) und das Erfassungsstück an dem Folienversiegelungsrahmen (73) befestigt sind, der Träger (72) auf dem Folienversiegelungsrahmen (73) in der zweiten horizontalen Richtung beweglich angeordnet ist und das Erfassungsstück in der Lage ist, einen Tragezustand des Trägers (72) zu erfassen.

7. Vorrichtung zur Verarbeitung von zu testenden Objekten nach Anspruch 5, wobei das Abdeckkörpertrageteil (721) mit einem ersten Begrenzungsvorsprung versehen ist, der dazu konfiguriert ist, den Abdeckkörper zu begrenzen, und/oder das Amplifikationsplattentrageteil (722) mit einem zweiten Begrenzungsvorsprung versehen ist, der dazu konfiguriert ist, die Amplifikationsplatte zu begrenzen.

8. Vorrichtung zur Verarbeitung von zu testenden Objekten nach Anspruch 1, die ferner einen Lagerbehälter (80) für Verbrauchsmaterial umfasst, der so konfiguriert ist, dass er eine Pipettenspitze trägt, wobei das Pipettierteil (60) mit einer Ladeposition versehen ist, in der sich das Pipettierteil (60) nach oberhalb des Lagerbehälters (80) für Verbrauchsmaterial bewegt, um die Pipettenspitze zu laden.

9. Vorrichtung zur Verarbeitung von zu testenden Objekten nach Anspruch 1, wobei das Pipettierteil (60) einen Probenarm (61) und eine Pipettiernadel (62) umfasst, wobei der Probenarm (61) horizontal beweglich am Rahmenkörper (10) angeordnet ist und sich oberhalb der Probenverarbeitungsplattform (11) befindet, und die Pipettiernadel (62) vertikal beweglich am Probenarm (61) angeordnet ist.

10. Vorrichtung zur Verarbeitung von zu testenden Objekten nach Anspruch 9, wobei der Rahmenkörper (10) mit einer ersten Führungsschiene (12) und einer zweiten Führungsschiene (13) versehen ist, die sich oberhalb der Probenverarbeitungsplattform (11) befinden, wobei sich die erste Führungsschiene (12) in der ersten horizontalen Richtung erstreckt, sich die zweite Führungsschiene (13) in der zweiten horizontalen Richtung erstreckt, die erste Führungsschiene (12) in einer Erstreckungsrichtung der zweiten Führungsschiene (13) beweglich unterhalb der zweiten Führungsschiene (13) angeordnet ist und der Probenarm (61) in einer Erstreckungsrichtung der ersten Führungsschiene (12) beweglich an der ersten Führungsschiene (12) angeordnet ist.

11. Vorrichtung zur Verarbeitung von zu testenden Objekten nach Anspruch 1, wobei das Greifteil (40) einen Greifrahmen und einen an dem Greifrahmen vertikal beweglich angeordneten Greifer umfasst, wobei der Greifrahmen an dem Rahmenkörper (10) in der ersten horizontalen Richtung beweglich angeordnet ist und der Greifer mit einer ersten Greifposition und einer zweiten Greifposition versehen ist.

12. Integrierte Maschine zum Testen von Nukleinsäuren, umfassend eine Reagenzvorbereitungsvorrichtung, eine Vorrichtung zur Verarbeitung von zu testenden Objekten und eine Amplifikationstestvorrichtung, wobei die Reagenzvorbereitungsvorrichtung so konfiguriert ist, dass sie ein Extraktionsreagenz und ein Amplifikationsreagenz vorbereitet, die Amplifikationstestvorrichtung so konfiguriert ist, dass sie eine Analytnachweisbestimmung an einem Bestimmungsgemisch durchführt, um ein zu testendes Objekt zu analysieren, und die Vorrichtung zur Verarbeitung von zu testenden Objekten die Vorrichtung zur Verarbeitung von zu testenden Objekten nach einem der Ansprüche 1-11 ist.

13. Integrierte Maschine zum Testen von Nukleinsäuren nach Anspruch 12, wobei,
die Reagenzvorbereitungsvorrichtung, die Vorrichtung zur Verarbeitung von zu testenden Objekten und die Amplifikationstestvorrichtung voneinander isoliert sind, sich die Reagenzvorbereitungsvorrichtung und die Amplifikationstestvorrichtung jeweils auf zwei Seiten der Vorrichtung zur Verarbeitung von zu testenden Objekten befinden, ein erster Kanal zwischen der Reagenzvorbereitungsvorrichtung und der Vorrichtung zur Verarbeitung von zu testenden Objekten angeordnet ist, ein zweiter Kanal zwischen der Vorrichtung zur Verarbeitung von zu testenden Objekten und der Amplifikationstestvorrichtung angeordnet ist, und der erste Kanal und der zweite Kanal einen Einschaltzustand und einen Ausschaltzustand aufweisen; und/oder,
die integrierte Maschine zum Testen von Nukleinsäuren ferner einen Fährmechanismus umfasst, der zwischen der Reagenzvorbereitungsvorrichtung und der Vorrichtung zur Verarbeitung von zu testenden Objekten angeordnet ist, wobei der Fährmechanismus in der Lage ist, eine Extraktionsplatte, die zum Vorbereiten des Extraktionsreagenzes konfiguriert ist, eine Amplifikationsplatte, die zum Vorbereiten des Amplifikationsreagenzes konfiguriert ist, und einen Abdeckkörper zu der Vorrichtung zur Verarbeitung von zu testenden Objekten durch den ersten Kanal zu bewegen, und das Greifteil (40) der Vorrichtung zur Verarbeitung von zu testenden Objekten in der Lage ist, die Extraktionsplatte zu einem Extraktionsplattentransferteil (53) der Vorrichtung zur Verarbeitung von zu testenden Objekten zu bewegen und die Amplifikationsplatte zu einem Amplifikationsplattentransferteil (54) der Vorrichtung zur Verarbeitung von zu testenden Objekten zu bewegen.

## Revendications

1. Appareil de traitement d'objets à tester, comprenant :
un corps de cadre (10), pourvu d'une plate-forme de traitement des échantillons (11) ;
un mécanisme d'extraction (30), configuré pour extraire un analyte dans un objet à tester ;
une pièce de préhension (40), mobile au-dessus de la plate-forme de traitement des échantillons (11) dans une direction verticale et une première direction horizontale ;
une pièce de transfert (50), disposée de manière mobile sur la plate-forme de traitement des échantillons (11) dans une seconde direction horizontale perpendiculaire à la première direction horizontale, la pièce de transfert (50) comprenant une pièce de transfert de plaque d'extraction (53), la pièce de transfert de plaque d'extraction (53) étant pourvue d'une première position de transfert où la pièce de transfert de plaque d'extraction (53) se déplace en dessous de la pièce de préhension (40), la pièce de préhension (40) étant pourvue d'une première position de préhension située au-dessus de la pièce de transfert de plaque d'extraction (53) et d'une deuxième position de préhension située au-dessus du mécanisme d'extraction (30), et lorsque la pièce de transfert de plaque d'extraction (53) est située dans la première position de transfert, la pièce de préhension (40) peut se déplacer entre la première position de préhension et la deuxième position de préhension pour déplacer une plaque d'extraction entre la pièce de transfert de plaque d'extraction (53) et le mécanisme d'extraction (30) ; et
une pièce de pipetage (60), disposée de manière mobile au-dessus de la plate-forme de traitement des échantillons (11) ;
dans lequel la pièce de transfert de plaque d'extraction (53) est en outre pourvue d'une position d'ajout d'échantillon correspondant à la pièce de pipetage (60), et lorsque la pièce de transfert de plaque d'extraction (53) est située dans la position d'ajout d'échantillon, la pièce de pipetage (60) est en outre pourvue d'une première position de pipetage où la pièce de pipetage (60) se déplace au-dessus de la pièce de transfert de plaque d'extraction (53).

2. Appareil de traitement d'objets à tester selon la revendication 1, dans lequel la pièce de transfert (50) comprend en outre une pièce de transfert de plaque d'amplification (54), dans lequel la pièce de transfert de plaque d'extraction (53) et la pièce de transfert de plaque d'amplification (54) se déplacent dans la seconde direction horizontale, la pièce de transfert de plaque d'amplification (54) est pourvue d'une position d'ajout d'acide nucléique au ras de la pièce de transfert de plaque d'extraction (53), la pièce de pipetage (60) est en outre pourvue d'une seconde position de pipetage correspondant à un côté supérieur de la pièce de transfert de plaque d'amplification (54), et lorsque la pièce de transfert de plaque d'extraction (53) est située dans la position d'ajout d'échantillon et que la pièce de transfert de plaque d'amplification (54) est située dans la position d'ajout d'acide nucléique, la pièce de pipetage (60) est commutée entre la première position de pipetage et la seconde position de pipetage.

3. Appareil de traitement d'objets à tester selon la revendication 2, dans lequel le mécanisme d'extraction (30) comprend une pièce d'extraction (31) et une pièce de transport (32) pénétrant de manière mobile à travers la pièce d'extraction (31) dans la seconde direction horizontale, dans laquelle la pièce de transport (32) est pourvue d'une position de transport correspondant à un côté inférieur de la pièce de préhension (40) et d'une position d'extraction située en dessous de la pièce d'extraction (31), et lorsque la pièce de transfert de plaque d'extraction (53) est située dans la première position de transfert et que la pièce de transport (32) est située dans la position de transport, la pièce de préhension (40) peut déplacer la plaque d'extraction entre la pièce de transfert de plaque d'extraction (53) et la pièce de transport (32).

4. Appareil de traitement d'objets à tester selon la revendication 2, comprenant en outre un mécanisme de scellage de film (70) configuré pour encapsuler une plaque d'amplification, dans lequel le mécanisme de scellage de film (70) comprend un support (72) et une pièce de pressage à chaud (71) configurée pour encapsuler la plaque d'amplification, dans lequel la pièce de pressage à chaud (71) est située au-dessus du support (72), le support (72) est disposé de manière mobile sur la plate-forme de traitement des échantillons (11) dans la seconde direction horizontale, et le support (72) est pourvu d'une pièce de transport de plaque d'amplification (722) configurée pour placer la plaque d'amplification ; la pièce de transfert de plaque d'amplification (54) est pourvue d'une seconde position de transfert située en dessous de la pièce de préhension (40) ; la pièce de préhension (40) est en outre pourvue d'une troisième position de préhension située au-dessus du support (72) et d'une quatrième position de préhension située au-dessus de la pièce de transfert de plaque d'amplification (54) ; et lorsque la pièce de transfert de plaque d'amplification (54) est située dans la seconde position de transfert, la pièce de préhension (40) peut se déplacer entre la troisième position de préhension et la quatrième position de préhension pour déplacer la plaque d'amplification de la pièce de transfert de plaque d'amplification (54) vers la pièce de transport de plaque d'amplification (722).

5. Appareil de traitement d'objets à tester selon la revendication 4, dans lequel le support (72) est en outre pourvu d'une pièce porteuse de corps de couverture (721) configurée pour placer un corps de couverture, lorsque la pièce de préhension (40) est située dans la troisième position de préhension, le support (72) est pourvu d'une position de séparation dans laquelle la pièce porteuse de corps de couverture (721) se déplace en dessous de la pièce de préhension (40) pour faire en sorte que la partie de préhension (40) saisisse le corps de couverture, une position de libération où la pièce porteuse de plaque d'amplification (722) se déplace sous la pièce de préhension (40) pour que la pièce de préhension (40) libère le corps de couverture sur la plaque d'amplification, et une position d'encapsulation où la pièce porteuse de la plaque d'amplification (722) se déplace sous la pièce de pressage à chaud (71).

6. Appareil de traitement d'objets à tester selon la revendication 5, dans lequel le mécanisme de scellage de film (70) comprend un cadre de scellage de film (73) et une pièce de détection, dans lequel le cadre de scellage de film (73) est disposé sur la plate-forme de traitement d'échantillons (11), la pièce de pressage à chaud (71) et la pièce de détection sont fixées au cadre de scellage de film (73), le support (72) est disposé de manière mobile sur le cadre de scellage de film (73) dans la seconde direction horizontale, et la pièce de détection peut détecter un état de portage du support (72).

7. Appareil de traitement d'objets à tester selon la revendication 5, dans lequel la pièce porteuse de corps du couvercle (721) est pourvue d'une première saillie de limitation configurée pour limiter le corps de couvercle, et/ou, la pièce porteuse de plaque d'amplification (722) est pourvue d'une seconde saillie de limitation configurée pour limiter la plaque d'amplification.

8. Appareil de traitement d'objets à tester selon la revendication 1, comprenant en outre un bac de stockage de consommables (80) configuré pour transporter une pointe de pipette, dans lequel la pièce de pipetage (60) est pourvue d'une position de chargement dans laquelle la pièce de pipetage (60) se déplace au-dessus du bac de stockage de consommables (80) pour charger la pointe de pipette.

9. Appareil de traitement d'objets à tester selon la revendication 1, dans lequel la pièce de pipetage (60) comprend un bras d'échantillon (61) et une aiguille de pipetage (62), dans lequel le bras d'échantillon (61) est disposé horizontalement et mobile sur le corps de cadre (10) et situé au-dessus de la plate-forme de traitement des échantillons (11), et l'aiguille de pipetage (62) est disposée verticalement et mobile sur le bras d'échantillon (61).

10. Appareil de traitement d'objets à tester selon la revendication 9, dans lequel le corps de cadre (10) est pourvu d'un premier rail de guidage (12) et d'un second rail de guidage (13) qui sont situés au-dessus de la plate-forme de traitement des échantillons (11), le premier rail de guidage (12) s'étend dans la première direction horizontale, le second rail de guidage (13) s'étend dans la seconde direction horizontale, le premier rail de guidage (12) est disposé de manière mobile en dessous du second rail de guidage (13) dans une direction d'extension du second rail de guidage (13), et le bras d'échantillon (61) est disposé de manière mobile sur le premier rail de guidage (12) dans une direction d'extension du premier rail de guidage (12).

11. Appareil de traitement d'objets à tester selon la revendication 1, dans lequel la pièce de préhension (40) comprend un cadre de préhension et une pince disposée verticalement et mobile sur le cadre de préhension, dans lequel le cadre de préhension est disposé de manière mobile sur le corps de cadre (10) dans la première direction horizontale, et la pince est pourvue d'une première position de préhension et d'une seconde position de préhension.

12. Machine intégrée de test d'acide nucléique, comprenant un appareil de préparation des réactifs, un appareil de traitement d'objet à tester et un appareil de test d'amplification, dans lequel l'appareil de préparation des réactifs est configuré pour préparer un réactif d'extraction et un réactif d'amplification, l'appareil de test d'amplification est configuré pour effectuer une détermination de détection d'analyte sur un mélange de détermination afin d'analyser un objet à tester, et l'appareil de traitement d'objets à tester est l'appareil de traitement d'objets à tester selon l'une quelconque des revendications 1 à 11.

13. Machine intégrée de test d'acide nucléique selon la revendication 12, dans laquelle,
l'appareil de préparation des réactifs, l'appareil de traitement d'objets à tester et l'appareil de test d'amplification sont isolés l'un de l'autre, l'appareil de préparation des réactifs et l'appareil de test d'amplification sont respectivement situés sur deux côtés de l'appareil de traitement d'objets à tester, un premier canal est disposé entre l'appareil de préparation des réactifs et l'appareil de traitement des objets à tester, un second canal est disposé entre l'appareil de traitement d'objets à tester et l'appareil de test d'amplification, et le premier canal et le second canal ont un état de marche et un état d'arrêt ; et/ou,
la machine intégrée de test d'acide nucléique comprend en outre un mécanisme de transfert disposé entre le dispositif de préparation des réactifs et l'appareil de traitement d'objets à tester, le mécanisme de transfert pouvant déplacer une plaque d'extraction configurée pour préparer le réactif d'extraction, une plaque d'amplification configurée pour préparer le réactif d'amplification, et un corps de couverture vers l'appareil de traitement d'objet à tester à travers le premier canal, et la pièce de préhension (40) de l'appareil de traitement d'objets à tester peut déplacer la plaque d'extraction vers une pièce de transfert de plaque d'extraction (53) de l'appareil de traitement d'objets à tester et de déplacer la plaque d'amplification vers une pièce de transfert de plaque d'amplification (54) de l'appareil de traitement d'objets à tester.
